# EUROPEAN PATENT SPECIFICATION

(11) **EP 2 397 161 B1**
(45) Date of publication and mention of the grant of the patent: **25.09.2019**
(21) Application number: 10741108.4
(22) Date of filing: 12.02.2010
(51) Int. Cl.: A61K 47/36, A61K 9/16, A61K 9/20, A61K 47/10, A61K 47/18, A61K 47/26

(54) **DISINTEGRATING PARTICLE COMPOSITION AND RAPIDLY DISINTEGRATING COMPRESSION-MOLDED MATERIAL COMPRISING SAME**
ZERFALLENDE TEILCHENZUSAMMENSETZUNG UND DIESE ENTHALTENDES SCHNELL ZERFALLENDES FORMGEPRESSTES MATERIAL
COMPOSITION DE PARTICULE SE DÉSINTÉGRANT ET MATÉRIAU MOULÉ PAR COMPRESSION SE DÉSINTÉGRANT RAPIDEMENT COMPRENANT CELLE-CI

(30) Priority: 12.02.2009 JP 2009029901
(43) Date of publication of application: 21.12.2011
(73) Proprietor: FUJI CHEMICAL INDUSTRY CO., LTD., Nakaniikawa-gun, Toyama 930-0397 (JP)
(72) Inventor: KOIZUMI, Haruka, Nakaniikawa-gun Toyama 930-0397 (JP); FUKAMI, Tadashi, Nakaniikawa-gun Toyama 930-0397 (JP); MACHIMURA, Hitoshi, Nakaniikawa-gun Toyama 930-0397 (JP)
(74) Representative: Adam, Holger
(86) International application number: PCT/JP2010/000886
(87) International publication number: WO 2010/092828

(56) References cited:
- WO-A1-01/39746
- WO-A1-01/76565
- WO-A1-97/15191
- WO-A1-98/01114
- WO-A1-2006/072832
- JP-A- 11 310 539
- JP-A- 2003 176 242
- JP-A- 2005 306 770
- JP-A- 2006 008 667
- JP-A- 2008 133 232
- JP-T- 2005 515 240
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

### Technical Field

This invention relates to a disintegrating particle composition which is obtained by dispersing a starch and a water soluble binding agent, and if needed, crystalline cellulose and inorganic fine particles uniformly in a solvent and then removing the solvent rapidly, and to a rapidly disintegrating compression-molded material comprising the same.

### Background Art

Intraoral rapid disintegrating tablets have been developed as the form which is easily taken by the aged, infants, and patients having difficulty in swallowing and which can be easily taken without using water. The intraoral rapid disintegrating tablets are required to solve many problems as compared with the ordinary tablets that the disintegration time in the oral cavity is within about 60 seconds, that they have a sufficient hardness for prevent them from causing cracking or dusting during the process of preparation, transport or opening of package containing them like the ordinary tablets, and that there is no sense of aversion in the taking feeling or taste in the oral cavity. Especially, disintegration time and hardness are conflicting factors each other and the maintenance of the both is most difficult.

Since the intraoral rapid disintegrating tablets require that the disintegration velocity is a short time, so-called super disintegrating agents such as polyvinyl pyrrolidone, low substituted hydroxypropylcellulose, sodium croscarmellose and the like have been employed. However, there were problems of side effect and regulation for taking them in a large amount. The intraoral rapid disintegrating tablets containing no these disintegrating agents have been studied.

An effervescent intraoral rapid disintegrating tablet is known, which comprises an organic acid, a carbonate salt, a network-maintaining agent such as corn starch and a coloring inhibitor such as maltose (Patent Literature 1). The organic acid and the carbonate salt are mixed together, compression-molded and heat-treated to cause a part of them to be reacted to form a porous structure while the remaining organic acid and carbonate salt effervesce in the oral cavity thereby the dissolution of the tablets in the oral cavity is increased. The existence of the organic acid and the heating process after the compression-molding are essential.

A rapid release compression-molded material obtained by subjecting an effective ingredient, maltose, corn starch, crystalline cellulose, silicon dioxide and other medicinal additive to dry blending and subjecting the mixture to kneading granulation, and thereafter subjecting the resultant granulated matter to compression-molding is known (Patent Document 2). Although there is a description regarding the release of the effective ingredient, there is no description regarding the disintegration in the oral cavity.

The present applicant proposed a rapidly disintegrating compression-molded material obtained by spray drying a homogeneous suspension of 0.5-99.5 % by weight of a saccharide such as erythritol and 0.5-99.5 % by weight of an inorganic compound such as magnesium aluminomethasilicate, blending the obtained spray dried composition with a starch or crystalline cellulose, and subjecting the resultant mixture to compression-molding (Patent Document 3). Furthermore, the present applicant proposed a composition for use in intraoral rapid disintegrating tablets, which is obtained by spray drying xylitol or other saccharide, an inorganic excipient and a disintegrating agent together and intraoral rapid disintegrating tablets comprises incorporated this composition (Patent Documents 4, 5 and 6). Although any of them has a structure of complex particles wherein other ingredients than the saccharide are dispersed in the structure wherein the main component is saccharide, there is no description regarding the composition containing starch as the main component being useful as an excipient for the intraoral rapid disintegrating tablets.

Although the preparation of them is simple and easy and the intraoral rapid disintegrating tablets have sufficiently excellent characteristics with respect to the hardness and the disintegration time, they need the incorporation of the disintegrating agent having a very high disintegrating property for achieving the desired disintegration, and care is required in the case that they are widely used in foods or pharmaceuticals. There has not been known a particle composition for use in the intraoral rapid disintegrating tablets having excellent taking feeling or disintegrating property wherein the main component is starches while it needs use of no disintegrating agent having a very high disintegrating property.
Patent Document 1: JP 11-310539 A
Patent Document 2: JP 2000-26292 A
Patent Document 3: JP 2000-86537 A
Patent Document 4: JP 2005-139168 A
Patent Document 5: WO 2005/37254 A
Patent Document 6: WO 2007/29376 A

WO 98/01114 A1 discloses a granulate, containing an active ingredient, having a solubility in water of 1:>10, in admixture with ≤15 wt.% of a water dispersible cellulose, for the preparation of fast-disintegrating and fast-dissolving compositions.

WO 2006/072832 A1 discloses a fast disintegrating dosage form of varenicline comprising an effective amount of varenicline or a pharmaceutically acceptable salt thereof and at least one pharmaceutically acceptable excipient, wherein the dosage form disintegrates in a patient's oral cavity in less than three minutes.

WO 97/15191 A1 relates to a pharmaceutical composition and a process for the preparation of a tablet containing a growth hormone secretagogue as the active ingredient.

WO 01/39746 A1 discloses superdisintegrants which provide improved compressibility compared to prior art superdisintegrants and which does not negatively impact the compressibility of formulations which include high-dose drugs, and methods for obtaining the same.

### Disclosure of Invention

### The Subject Matter to be Solved by the Invention

An object of the invention is to provide an intraoral rapid disintegrating tablet which contains no disintegrating agent having high disintegrating property, which has good hardness, disintegrating property and taking feeling and which can be used widely in the fields of foods and pharmaceuticals.

Another object of the present invention is to provide an excipient for use in the intraoral rapid disintegrating tablets and which is necessary for preparing the tablets. The Means to Solve the Subject Matter

As a result of having ardently studied to achieve the above-described objects, the present inventors have found that there can be obtained an intraoral rapid disintegrating tablet which has an excellent disintegrating property and which has a sufficient hardness for preventing the tablet from causing any problem during the preparation or transport and taking feeling and taste not to cause any problem in the oral cavity by blending a particle composition with an effective ingredient and additives such as an excipient, a disintegrating agent, a binding agent and a lubricant together, said particle composition being one obtained by dispersing a starch and a water soluble binding agent, and if needed crystalline cellulose or inorganic fine particles uniformly in a solvent and removing the solvent rapidly.

The object is solved by the features disclosed in the independent claims. Preferred embodiments are disclosed in the dependent claims.

### Effect of the Invention

Since an excipient for use in the intraoral rapid disintegrating tablet in the present invention comprises components each is taken without limitation in quantity, it can be used in the preparation of the intraoral rapid disintegrating tablets for foods and pharmaceuticals which can be taken in a large amount without taking care about the taking amount.

### Brief Description of the Drawing

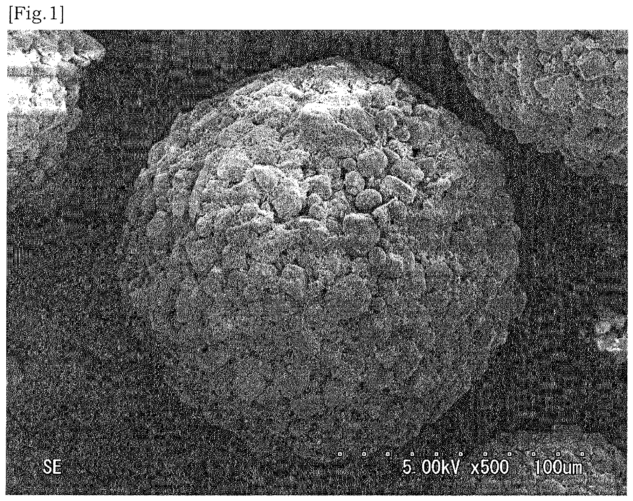

[Fig.1]:SEM photograph (one memory: 10 µm) of the spray-dried particles in Example 6.

### Best Mode for Carrying Out the Invention

In the particle composition of the present invention, complex particles are formed by dispersing a starch, a water soluble binding agent, and if needed a molding agent such as crystalline cellulose or inorganic particles and/or an effective ingredient uniformly in a solvent and removing the solvent rapidly.

The complex particles in the present invention have a structure wherein insoluble particles such as a starch and the like are singly and uniformly dispersed in the matrix of a water-soluble binding agent. By taking the structure of the complex particles, the surface of starch is coated completely thickly without void with the water-soluble binding agent to improve the quality so as to hold disintegrating property of starch while the molding property is increased (the molding pressure is lowered) and particular powdery feeling (swelling powder feeling) is improved. Small particles coagulate each other to form secondary particles. The characteristic feature of the present invention is that a starch is singly dispersed up to apparent primary particles. The single dispersion herein refers to the same level of dispersion degree as that in the dispersion treatment of the pretreatment taken in the measurement of particle size distribution by wet process.

Examples of starches in the present invention include starches derived from grain such as corn starch, rice starch, waxy corn starch, high amylose corn starch, wheat starch, glutinous rice starch and the like; starches derived from bean such as broad bean starch, mung bean starch, adzuki bean starch, soybean starch, pinto bean starch, red kidney bean starch and the like; starches derived from tubers or roots such as potato starch, sweet potato starch, tapioca starch, taro starch, dogtooth violet starch and the like.

These starches may be adequately used in combination thereby the characteristics of the particle composition involving in the present invention may be adjusted.

Of these starches, a starch containing 1 % by weight or less of amylose (in the case where it was added to water, viscosity does not occur) may be mixed with a starch containing 15 -30 by weight % of amylose (in the case where it was added to water, viscosity tends to occur). General starch contains a small amount of amylose and causes powdery feeling in the taking feeling of the oral cavity. Contrary thereto, starch having high amylose content adsorbs water to cause viscosity, and therefore it may improve powdery feeling of insoluble powders. As starch having high amylose content, glutinous rice starch and waxy corn starch are taken. As starch having low amylose content, there are taken other starches than them. The compounding ratio of starch having low amylose content to starch having high amylose is 1:0.05-20, preferably 1:0.1-12.

As a water-soluble binding agent in the present invention, among ingredients which may be incorporated into foods and which may be taken in a large amount, water-soluble saccharides and amino acids are taken. Saccharides include saccharide and saccharide alcohol. Examples of saccharides include xylitol, erythritol, maltitol, sorbitol, mannitol lactose, sucrose, glucose, fructose, maltose, trehalose, palatinose. Examples of amino acids include glycine, alanine, serine, lysine, threonine, cystine, hystidine, asparagine, aspartic acid, glutamine and glutamic acid. These saccharides and amino acids may be used singly or in combination of two or more kinds.

The molding agent is used for preparation of the tablets having sufficient hardness even under low molding pressure (for enhancing molding property) because in the case of the particle composition containing a starch as the main component the molding pressure during the compression-molding become high. As examples of molding agents, crystalline cellulose, inorganic particles and the like may be taken.

The inorganic particles are fine ones having an average particle size of 0.1-300 µm and high specific surface area. The use of them causes effect of lowering in the molding pressure during compression molding of the granulated particles of the present invention, water-inducting effect in the oral cavity and effect of the increase in the yield during the preparation of granulated particles. Among inorganic substances having these physical properties, for example, magnesium carbonate, calcium carbonate, magnesium aluminomethasilicate, magnesium aluminosilicate, calcium hydrogen phosphate, anhydrous calcium hydrogen phosphate, anhydrous calcium hydrogen phosphate granulated matter, hydrotalcite, aluminum silicate, calcium phosphate, calcium silicate, magnesium silicate, magnesium oxide, magnesium hydroxide, magnesia alumina hydrate, dry aluminum hydroxide gel and the like are taken. Among them, magnesium carbonate, calcium carbonate, anhydrous silicic acid, calcium silicate, calcium hydrogen phosphate and anhydrous calcium hydrogen phosphate are preferred.

The terms "high specific surface area" refer to BET specific surface area having 5-1000 g/m², preferably 10-500 g/m². Since inorganic particles having high specific surface area have a aggregated structure of primary particle having several hundred nm or less, it is considered that their permeability in the granulated particles may be enhanced by water-inducting effect in the granulated matter. Also, in the case that a suspended solution of ingredients is spray-dried, which may be used in the preparation of the particle composition of the present invention, they cause effects to suppress aggregation among particles to enhance the productivity of the granulated matter. At the same time, they cause a secondary effect that the moldability is enhanced.

In the powder composition comprising starch and a water-soluble binding agent, the ratio by weight of starch to water-soluble binding agent is 1:0.01-0.75, preferably 1:0.03-0.3, most preferably 1:0.05-0.2. In the case that the powder composition contains further a molding agent, the ratio by weight of starch to the molding agent is 1:0.01-1, preferably 1:0.1-0.5, most preferably 1:0.15-0.4.

In the case that crystalline starch and inorganic particles are incorporated as a molding agent in the particle composition, the ratio by weight of crystalline starch to the inorganic particles is 1:0.1-10, preferably 1:0.2-2, most preferably 1:0.4-1.2.

An average particle size of the particle composition in the present invention may be 1-500 pm, preferably 5-300 µm, more preferably 10-200 µm from the prevention of rough sensation in the oral cavity and disintegrability. The sphericity may be 0.7 or more preferably 0.8 or more from the compression-moldability and disintegrability. From these characteristic properties, the particle composition is particle having a good fluidity and hence it has an excellent tableting property during tableting process.

A static specific volume of the particle composition in the present invention may be 1.5-4.0 g/ml, preferably 1.5-3.5 g/ml, more preferably 1.5-2.5 g/ml. Since the particle composition has such a static specific volume, in the case that it is formed into tablets it can be easily filled into a mortar so that the tableting process proceeds smoothly. Also, it can be compressed uniformly to provide an excellent tableting property. The static specific volume can be measured according to the standard method.

There can be incorporated into the powder composition during its preparation two or more of effective ingredients and other ingredients which do not injure the disintegrating property of the product and which are used in the pharmaceuticals. The amount incorporated of the effective ingredient may be 0.1-100 parts by weight, preferably 0.1-80 parts by weight, more preferably 0.1-50 parts by weight based on 100 parts by weight of the total amount of a starch and a water-soluble binding agent.

The powder composition of the present invention may be prepared by a wet process for obtaining the desired physical properties and the generally used process, for example wet granulating processes such as spray drying process, fluidized bed granulating drying process, agitated granulating process, wet extruding granulating process, etc. may be applied. For removing a solvent rapidly, a process wherein a dispersion solution may be instantaneously dried using a spray nozzle is preferred. Fluidized bed granulation, agitated granulation and tumbling granulation where a dispersed solution is sprayed to nuclear particles for granulation, and spray drying where granulation is conducted only by spraying process of a dispersed solution are preferred. However, spray drying process is most preferable from the viewpoints that the preparation process is easy and the desired physical properties may be easily obtained.

In the wet granulating process, by setting condition a starch, a water-soluble binding agent, and if needed crystalline cellulose or inorganic fine particles and other ingredients are uniformly dispersed in a solvent and then the solvent is removed rapidly thereby obtaining the particle composition of the present invention. By this preparation process, for forming the structure of the complex particles where poorly soluble particles are uniformly dispersed in the matrix of the water-soluble binding agent as explained already, a water-soluble binding agent is dissolved in a dispersing solvent and its layer is formed on the periphery of the poorly soluble particles, and the particles involving in the particle composition of the present invention are aggregated one another to form clean and spherical particles. The condition may be set so as to meet the characteristics of wet granulating machine.

As the preparation process based on spray drying process, a starch, a water-soluble binding agent, and if needed a molding agent or inorganic particles are dispersed in an aqueous solvent, and then the resultant dispersion is subjected to spray drying according to the conventional manner thereby obtaining the particle composition of the present invention. More specifically, a starch, a water-soluble binding agent, and if needed a molding agent or inorganic particles are added to an aqueous solvent to prepare a dispersed solution. The addition order of these ingredients is not related particularly. In the case that some ingredients are intended to be dissolved completely, it is preferable that a water-soluble binding agent is previously dissolved or dispersed in an aqueous solvent, and thereafter a starch, a molding agent and a sparingly soluble substance such as inorganic particles are uniformly dispersed to prepare a dispersed solution. Consequently, this dispersed solution is subjected to spray drying thereby there can be obtained the particle composition of the present invention. Also, one or more of effective ingredients and other ingredients which do not injure the disintegrating property of the product and which are used in pharmaceuticals are further dispersed uniformly in the dispersed solution and the resultant dispersed solution may be subjected to spray drying for the preparation of the particle composition.

The above-described solvent may be one which does not affect on the characteristics of the powder composition and which is permissible in pharmaceuticals or foods. For example, water, ethanol and the like are taken and they may be used in combination. The dispersed solution may be prepared by the known process. Although usual agitation, colloid mill, high-pressure homogenizer, ultrasonic irradiation and the like are taken, it may be any process wherein particles can be dispersed up to high degree in an aqueous dispersed solution. As to the concentration of the ingredients in the dispersed solution, it may be such an extent that spray drying may be conducted without being affected by the viscosity of the dispersed solution or the like. That is, it may be 5-50 % by weight, preferably 10-45 % by weight.

Although the condition for spray drying is not restricted particularly, the use of disk or nozzle type of spray drying machine is preferable. And, as to the temperatures at which spray drying is conducted, it is preferable that an input temperature is about 120-220 °C while an output temperature is about 80-130 °C. As to the concentration of the solid substance in an aqueous dispersed solution during spray drying, it may be such an extent that spray drying may be conducted. The particle size of the particle composition may be adequately adjusted depending on the concentration of the aqueous solution or aqueous dispersed solution, spray drying method, drying condition and the like.

As the preparation process based on fluidized bed granulation, agitated granulation and tumbling granulation, insoluble ingredients such as a starch, crystalline cellulose, anhydrous calcium hydrogen phosphate, magnesium aluminomethasilicate are acted as nuclear particles while other ingredient-dispersed solution is sprayed thereby obtaining the particle composition of the present invention. Partially insoluble ingredients may be further suspended in the dispersed solution and sprayed. The preparation condition may be set so as to form the structure of complex particles in the particle composition of the present invention as aforementioned. For example, the drying velocity is made slow by increasing the amount of spraying solvent and lowering the temperature so that the suspension becomes affinity with nuclear particles to be uniformly dispersed.

The terms "rapid disintegrating compression-molded material" in the present invention mean tablet which disintegrates rapidly in the oral cavity, e.g., within 60 seconds, preferably 40 seconds, more preferably within 30 seconds. The disintegration time in the oral cavity referred to herein is time obtained by the condition in the intraoral rapid disintegrating tablet or the process in Examples described later. The disintegration time in the oral cavity may be varied depending on the size and shape of a tablet, but such variation is also within a scope of the present invention.

The terms "taking feeling not to cause any unplesant feeling in the oral cavity" mean one not to provide powder feeling, muddy odor or swelling powder feeling being increased when the incorporated raw material absorbes water. And, the terms "taste not to cause any unplesant feeling in the oral cavity" mean one not to have acidity (sourness), bitterness, astringency or the like derived from the raw material.

An effective ingredient, the coated effective ingredient or granulated effective ingredient can be added to the composition to be compression-molded. Since the molding or disintegrating property varies by their inherent properties, an excipient, a disintegrating agent and a binding agent may be added for adjusting the molding or disintegrating property. They may be added to the particle composition or to the composition to be compression-molded. The addition of them to the composition to be compression-molded is that they are mixed with the powder composition and an effective ingredient and the like together and then compression-molded. Incidentally, an excipient, a disintegrating agent and a binding agent are contained in other ingredients which do not cause the disintegrating property of the product and which are used in pharmaceuticals.

The rapid disintegrating compression-molded material of the present invention may contain if needed at least one material selected from among an effective ingredient, an excipient, a disintegrating agent, a binding agent, a surfactant, a lubricant, an acidifier, a sweetener, a corrigent , a perfume, a coloring agent and a stabilizer. As to the amount incorporated of the respective materials, 0.01-1000 parts by weight of at least one material selected from among an excipient, a disintegrant aid, a binder aid, a surfactant, a lubricant, an acidifier, a sweetener, a corrigent , a perfume, a coloring agent, a stabilizer and an effervescent, and if needed 0.1-100 parts by weight of an effective ingredient are incorporated based on 100 parts by weight of the particle composition.

More specifically, 1-99 parts by weight of the particle composition, 0.01-5 parts by weight of a lubricant, 1-99 parts by weight of at least one material selected from among an excipient, a disintegrating agent and a binding agent and 0.01-60 parts by weight of an effective ingredient are incorporated based on 100 parts by weight of the whole tablet. Preferably, 10-90 parts by weight of the particle composition of the present invention, 0. 1-3 parts by weight of a lubricant, 10-90 parts by weight of at least one material selected from among an excipient, a disintegrant aid and a binder aid and 0.01-50 parts by weight of an effective ingredient are incorporated based on 100 parts by weight of the whole tablet.

A lubricant, an excipient, a disintegrating agent, and/or a binding agent may not mixed together immediately before the preparation of tablets, and they may be previously mixed with the particle composition of the present invention. It is especially preferable that excipients such as the aforementioned starches are previously mixed with the particle composition for making pharmaceutical process such as the fluidity of the particle composition good.

Examples of lubricants for use in the present invention include powdered gum Arabic, cocoa butter, carnauba wax, calcium carmellose, sodium carmellose, caro peptide, hydrated silicon dioxide, dry aluminum hydroxide gel, glycerin, magnesium silicate, light anhydrous silicic acid, light liquid paraffin, crystalline cellulose, hydrogenated oil, synthetic aluminum silicate, sesame oil, wheat starch, white beeswax, magnesium oxide, dimethyl polysiloxane, sodium potassium tartrate, sugar fatty acid ester, glycerin fatty acid ester, silicone resin, aluminum hydroxide gel, stearyl alcohol, stearic acid, aluminum stearate, calcium stearate, polyoxyl stearate, magnesium stearate, cetanol, gelatin, talc, magnesium carbonate, precipitated calcium carbonate, corn staech, lactose, hard fat, white soft sugar, potato starch, hydroxypropylcellulose, fumaric acid, sodium stearyl fumarate, polyethylene glycol, polyoxyethylene-polyoxypropylene glycol, polysorbate, beeswax, magnesium aluminomethasilicate, methylcellulose, Japan wax, glyceryl monostearate, sodium lauryl sulfate, calcium sulfate, magnesium sulfate, magnesium sulfate, liquid paraffin, phosphoric acid and the like. Among them, stearic acid, magnesium stearate, calcium stearate, sugar fatty acid ester, glycerin fatty acid ester, polyoxyethylene glycol, sodium stearyl fumarate, talc and hydrogenated oil are preferred.

Examples of excipients in the present invention include the aforementioned starches, starch acrylate, L-aspartic acid, aminoethyl sulfonic acid, aminoacetic acid, barley sugar (powder), gum arabic, powdered gum arabic, alginic acid, sodium alginate, pregelatinized starch, inositol, ethylcellulose, ethylene-vinyl acetate copolymer, erythritol, sodium chloride, olive oil, kaolin, cocoa butter, casein, fructose, pumice grains, carmellose, sodium carmellose, hydrated silicon dioxide, dry yeast, dry aluminum hydroxide gel, dry magnesium sulfate, agar, powered agar, xylitol, citric acid, sodium citrate, disodium citrate, glycerin, carcium glycerophosphate, sodium gluconate, L-glutamine, clay, clay grain, sodium cross carmelose, aluminum silicate, synthetic aluminum silicate, magnesium aluminosilicate, calcium silicate, magnecium silicate, light anhydrous silicic acid, light liquid paraffin, powdered cinnamon, crystalline cellulose, crystalline cellulose-sodium carmellose, microcrystalline cellulose, brown rice malt, synthetic aluminum silicate, synthetic hydrotalcite, sesame oil, wheat flour, wheat starch, wheat germ powder, rice flour, rice starch, potassium acetate, calcium acetate, cellulose acetate phthalate, safflower oil, white beeswax, zinc oxide, titanium oxide, magnesium oxide, β-cyclodextrin, dihydroxy aluminum aminoacetate, 2,6-di-t-buthyl-4-methylphenol, dimethyl polysiloxane, tartaric acid, potassium hydrogen tartrate, exsiccated gypsum, sugar fatty acid ester, magnesia alumina hydrate, aluminum hydroxide gel, aluminum hydroxide-sodium hydrogen carbonate co-precipitate, magnesium hydroxide, squalane, stearyl alcohol, stearic acid, calcium stearate, polyoxyl stearate, magnesium stearate, purified gelatin, purified shellac, purified white soft sugar, purified white soft sugar spherical granule, purified montan wax, zein, sorbitan sesquioleate, cetanol, gypsum, cetostearyl alcohol, shellac, gelatin, sorbitan fatty acid ester, D-sorbitol, tribasic calcium phosphate, soybean oil, unsaponifiable matter of soybean oil, soybean lecithin, powdered skim milk, talc, ammonium carbonate, calcium carbonate, magnesium carbonate, neutral anhydrous sodium sulfate, low substituted hydroxypropylcellulose, dextran, dextrin, natural aluminum silicate, corn syrup, corn starch, trehalose, traganth, silicon dioxide, calcium lactate, lactose, hydrotalcite, maltose, white shellac, white petrolatum, white clay, white soft sugar, white soft sugar starch spherical granule, wheat green leaf extract powder, powdered wheat green leaf juice, honey, palatinose, paraffin, potato starch, semi-digested starch, human serum albumin, hydroxypropyl starch, hydroxypropyl cellulose, phytic acid, glucose, glucose hydrate, partly pregelatinized starch, pullulan, propylene glycol, reduced maltose syrup powder, powd ered cellulose, pectin , bentonite, sodium polyacrylate, polyethylene glycol, polyoxyethylene alkyl ether, polyoxyethylene hydrogenated caster oil, polyoxyethylene polyoxypropylene glycol, sodium polystyrene sulfonate, polysorbate, polyvinylacetal diethylaminoacetate, polyvinyl pyrrolidone, maltitol, maltose, D-mannitol, sugar syrup, isopropyl myristate, anhydrous lactose, anhydrous calcium hydrogen phosphate, anhydrous calcium hydrogen phosphate granulated matter, magnesium aluminometasilicate, methylcellulose, cotton seed meal, cotton seed oil, Japan wax, aluminum monostearate, glyceryl monostearate, sorbitan monostearate, anhydrous silicic acid, medicinal carbon, peanut oil, aluminum sulfate, calcium sulfate, granular limestone, granular corn starch, liquid paraffin, dl-malic acid, calcium phosphate, calcium hydrogen phosphate, potassium hydrogen phosphate and sodium hydrogen phosphate. They may be used singly or in combination. Among them, crystalline cellulose, sodium croscarmellose, magnesium aluminomethasilicate, magnesium aluminosilicate, calcium hydrogen phosphate, anhydrous calcium hydrogen phosphate, anhydrous calcium hydrogen phosphate granulated matter, calcium silicate, aluminum silicate, anhydrous silicic acid and magnesium carbonate are preferred.

Examples of disintegrating agents in the present invention include the aforementioned starches, adipic acid, alginic acid, sodium alginate, pregelatinized starch, erythritol, sodium carboxtmethyl starch, carmellose, calcium carmellose, sodium carmellose, hydrated silicon dioxide, agar, xylitol, guar gum, calcium citrate, sodium croscarmellose, crospovidone, synthetic aluminum silicate, magnesium aluminosilicate, low substituted hydroxypropyl cellulose, crystalline cellulose, crystalline cellulose-sodium carmellose, wheat starch, rice starch, cellulose acetate phthalate, dioctyl sodium sulfosuccinate, sugar fatty acid ester, magnesia alumina hydrate, calcium stearate, polyoxyl stearate, sorbitan sesquioleate, gelatin, shellac, sorbitol, sorbitan fatty acid ester, talc, sodium hydrogen carbonate, magnesium carbonate, precipitated calcium carbonate, dextrin, dehydroacetic acid, corn starch, traganth, trehalose, lactose, maltose, white soft sugar, hydrotalcite, honey, palatinose, hydroxyethylmethyl cellulose, hydroxtpropyl starch, glucose, bentonite, partly pregelatinized starch, monosodium fumarate, polyethylene glycol, polyoxyethylene hydrogenated caster oil, polyoxyethylene-polyoxyepropylene glycol, polysorbate, polyvinylacetal diethylaminoacetate, polyvinyl pyrrolidone, maltinol, D-mannitol, anhydrous citric acid, anhydrous silicic acid, magnesium alminomethasilicate, methylcallulose, glycerin monostearate, sodium lauryl sulfate and the like. Any of them may be used singly, but they may be incorporated in combination. Among them, low substituted hydroxypropyl cellulose, crystalline cellulose, carmellose, sodium croscarmellose, sodium carboxymethyl starch, rice starch, corn starch, crospovidone, magnesium alminomethasilicate, magnesium alminosilicate and synthetic aluminum silicate are preferred.

Examples of binding agents in the present invention include alginic acid, ethyl acrylate-methylmethacrylate copolymer, acetyl glycerin fatty acid ester, aminoalkylmethacrylate copolymer E, aminoalkylmethacrylate copolymer RS, aminoethylsulfonic acid, sugar syrup (powder), gum arabic, powdered gum arabic, sodium alginate, alginic acid propylene glycol ester, pregelatinized starch, ester gum H, ethylcellulose, powdered phellodendron bark, hydrolyzed gelatin powder, sodium caseinate, fructose, caramel, gum karaya powder, carboxyvinyl copolymer, carboxtmethylethylcellulose, sodium carboxtmethyl starch, carmellose, sodium carmellose, hydrated silicon dioxide, agar, kanbaiko, xanthan gum, beef tallow hydrogenated oil, guar gum, glycerin, synthetic aluminum silicate, light anhydrous silicic acid, light anhydrous silicic acid-containing hydroxypropyl cellulose, crystalline cellulose, hydrogenated oil, copolividon, sesame oil, wheat flour, wheat starch, rice flour, rice starch, vinylacetate resin, cellulose acetate phthalate, white beeswax, oxidized starch, dioctyl sodium sulfosuccinate, dihydroxyaluminum aminoacetate, sodium potassium tartrate, sugar fatty acid ester, stearyl alcohol, stearic acid, calcium stearate, polyoxyl stearate, sorbitan sequioleate, cetanol, gelatin, shellac, sorbitan fatty acid ester, D-sorbitol, soybean lecithin, calcium carbonate, simple syrup, dextrin, starch (soluble), corn starch, traganth, paraffin, potato starch, hydroxyethyl cellulose, hydroxyethylmethylcellulose, hydroxypropyl starch, hydroxypropylcellulose, hydroxypropylmethylcellulose, hydroxypropylmethylcellulose acetate succinate, hydroxypropylmethylcellulose phthalate, piperonyl butoxide, butyl phthalyl butyl glycolate, glucose, partly pregelatinized starch, fumaric acid, pullulan, propylene glycol, pectin, sodium polyacrylate, polyacrylic acid partially neutralized matter, polyethylene glycol, polyoxyethlene polyoxypropylene glycol, polysorbate, polyvinylacetal diethylaminoacetate, polyvinyl alcohol, (completely saponifized matter), polyvinyl alcohol, (partially saponifized matter), polyvinyl pyrrolidone, polybuten, sodium polyphosphate, D-mannitol, sugar syrup, light anhydrous silicic acid, sodium aluminomethasilicate and the like. Although any of them may be used singly, they may be incorporated in combination.

The effective ingredient is not restricted particularly, and it includes peripheral nervous system agent, antipyretic, analgesic anti-inflammatory agent, central nervous system drugs such as hypnic sedative, psychic and nervous system agent, etc.; peripheral nervous system agents such as skeletal muscle relaxants, autonomic nervous system agents, etc.; cardiovascular and circulatory system agents such as cardiotonics, anti-arrhythmic drugs, diuretics, vasodilators; respiratiry system agents such as bronchodilators, antitussives, etc.; gastrointestinal system agents such as digestive, intestinal function-controlling agent, antacids, etc.; metabolic drugs such as hormones, antihistaminic agents, vitamins, etc.; anti-ulcer agents, antibiotics, chemotherapeutic agents, crude drug-extracts, microorganisms and the like.

There may be taken an active agent for cold remedy, an active agent for rhinitis and the like. Examples of the active agent for cold remedy include antipyretic, analgesic anti-inflammatory agents, vasodilators, antihistaminic agents, antitussives, expectorants, antitussive expectorants, vitamins, Chinese medicine extracts and the like. Examples of an active agent for rhinitis include sympathomimetics, sympatholytics, anti-allergenic agents, anti-inflammatory agents and the like. Examples of antipyretic, analgesic anti-inflammatory agents include aniline derivatives such as pranluskast hydrate, acetaminophen, phenacetin, leferamine hydrochloride and the like; salicylic acid derivatives such as ethenzamide, sasapyrine, methyl salicylate, phenyl salicylate, sodium salicylate, choline salicylate, aspirin, aspirin aluminum and the like; pyrazolone derivatives such as isopropylantipyrine, sulpyrin, phenylbutazone, ketophenylbutazone, antipyrine, aminopyridine and the like; propionic acid derivatives such as ibuprofen, ketoprofen, meloxicam, oxaprozin, naproxen, fenoprofen calcium, tiaprofenic acid and the like; phenylacetic acid derivatives such as fenbufen, diclofenac sodium, anfenac sodium and the like; indoleacetic acid derivatives such as indomethacin, indomethacin farnesil, proglumetacin maleate, sodium tolmetin and the like; anthranilinoacetic acid derivatives such as mefenamic acid, flufenamic acid, tolfenamic acid and the like; oxicam derivatives such as piroxicam, ampiroxicam, tenoxicam and the like; benzydamine hydrochloride, epirizole (mepirizole), tinoridine hydrochloride, tiaramide hydrochloride, etc., anti-inflammatory enzyme preparations, serrapeptase (trade name), lysoxyme chloride, celecoxib and the like. These antipyretic, analgesic anti-inflammatory agents may be used singly or in combination.

Examples of bronchodilators include ephedrine hydrochloride, dl-methylephedrine hydrochloride, dl-methylephedrine hydrochloride saccharinate, isoprenaline hydrochloride, isoproterenol sulfate, methoxyphenamine hydrochloride, orciprenaline sulfate, chlorprenaline hydrochloride, trimetoquinol hydrochloride, salbutamol sulfate, terbutaline sulfate, hexoprenaline sulfate, formoterol fumarate, fenoterol hydrobromide, procaterol hydrochloride, pirburol hydrochloride, clenbuterol hydrochloride, mabuterol hydrochloride, xanthine derivatives such as aminophylline, theophylline, diprophylline, proxyphylline and the like; anti-cholinergics such as flutropium bromide, oxitropium bromide and the like. Examples of antihistaminic agents include ethanolamine antihistaminic agents such as diphenhydramine and the like; propylamine antihistaminic angents such as dl-chloropheniramine maleate, d-chloropheniramine maleate and the like; phenothiazine, montelukast antihistaminic agents such as alimemazine tartrate, isothipendyl hydrochloride, promethazine hydrochloride, mequitazine, and the like; diphenylpyraline, carbinoxamine maleate, clemastine fumarate, iproheptine hydrochloride, homochlorcyclizine hydrochloride, cycloheptazine hydrochloride, dimetindene maleate, triprolidine hydrochloride, olopatadine hydrochloride, carbocisteine, salmeterol xinafonate, fluticasone propionate, montelukast sodium, tiotropium bromide hydrate, cetirizine hydrochloride and the like.

Examples of antitussives include codeines such as codeine phosphate, dihydrocodeine phosphate and the like; dextromethorphan hydrobromide, cloperastine, noscapine dimemorfan, oxeladin, pentoxyverine citrate, eprazinone hydrochloride, clobutinol hydrochloride, isoaminil citrate, fominoben hydrochloride, clobedanol hydrochloride, benproperine phosphate, hydrocotarnine hydrochloride, sodium dibunate, etc.

Examples of expectorants include potassium guaiacolsulfonate; cysteine derivatives such as carbocysteine, L-ethylcysteine hydrochloride, L-methylcysteine hydrochloride, acetylcysteine and the like; bromhexine, ambroxol hydrochloride, etc. Examples of antitussive expectorants include guaifenesin, tipepidine, oximethebanol, alloclamide hydrochloride, carbetapentane citrate, trimetoquinol hydrochloride, methoxyphenamine hydrochloride and the like. Some of the pharmaceutically active ingredients exemplified as anthitussives, expectorants and antitussive expectorants described above show antitussive and/or expectorant actions.

Examples of psychotropic drugs include chlorpropamazine, reserpine, olanzapine, risperidone, quetiapine fumarate, aripiprazole and the like. Examples of antianxieth agent include tofisopam, zolpidem tartrate, alprazolam, chlordiazepoxide, diazepam, etizolam, quetiapine fumarate and the like. Examples of antidepressants include tricyclic medicine such as imipramin and the like; tetracyclic medicine such as maprotiline hydrochloride and the like, SSRI (selective serotonin reuptake inhibitor) such as sertraline hydrochloride, fluvoxamine maleate, escitalopram and the like; SNRI (serotonin noradrenaline reuptake inhibitor) such as milnacipran hydrochloride, venlafavine, duloxetine and the like. Examples of hypnic sedatives include estazolam, nitrazepam, diazepam, perlapine, phenobarbital sodium and the like. Examples of spasmilytics include scopolamine hydrobromide, papaverine hydrochloride, diphenhydramine hydrochloride and the like. Examples of central nervous system acting drugs include citicoline and the like. Examples of antiepileptics include phenytoin, carbamazepine, sodium valproate, topiramate, lamotrigine and the like. Examples of sympathomimetic agent include isoproterenol and the like. Examples of peripheral neuropathy drug include epalrestat, mecobalamine and the like.

Examples of gastrointestinal drugs include stomachics and digestives such as diastase, saccharated pepsin, scopolia extract, cellase AP 3, lipase AP, cinnamon oil, mesapride citrate; medicines for intestinal didorders such as berberine chloride, lactobacillus resistant bifidus and the like. Examples of antacids include magnesium carbonate, sodium hydrogen carbonate, synthetic hydrotalcite, precipitated calcium carbonate, magnesium oxide and the like. Examples of antiulcer drugs include teprenone, farmotidine, lansoprazole, omeprazole, rabeprazole, cimetidine rantidine hydrochloride, esomeprazole, pantoprazole and the like.

Examples of anti-hypertensive agents include carvediol, olmesartan medoxomil, benidipine hydrochloride, telmisartan, amlodipine besylate, delapril hydrochloride, captopril, hydralazine hydrochloride, labetralol hydrochloride, manidipine hydrochloride, candesartan cilexetil, methyldopa, perindopril erbumin, bisoprolol fumarate, valsartan, losartan potassium, irbesartan and the like. Examples of vasoconstrictors include phenylephrine hydrochloride and the like. Examples of vasodilators include nicorandil, carbocromen hydrochloride, morsidomine, verapamil hydrochloride, cinnarizine, diltiazem hydrochloride , cilnidipine, imidapril hydrochloride and the like. Examples of antiarrhythmic agents include procainamide hydrochloride, propranolol hydrochloride, pindolol, pilsicainide hydrochloride and the like. Examples of cardiotomics include caffeine, digoxin and the like. Examples of diuretics include isosorbide, furosemide, hydrochrothiazide and the like. Examples of agents for hyperlipidemics include ethyl icosapentate, sodium cerivastatin, simvastatin, sodium pravastatin, calcium pitavastatin, calcium atorvastatin hydrate, bezafibrate, calcium rosuvastatin, ezetimibe, combined medicine such as combination of ezetimibe and simvastatin and the like.

Examples of antiobiotics include vancomycine hydrochloride, cefdinir, itraconazole, clarithromycine, cephem antiobiotics such as cefcapene pivoxil hydrochloride, cefalexin, cefaclor, amoxicillin, pipumeshirinamu hydrochloride, cefotiam hexetil hydrochloride, cefadroxil, cefixime, cefditoren pivoxil, cefteram pivoxil, cefpodoxime proxetil; synthetic bactercides such as ampicillin, cyclacillin, nalidixic acid, levofloxacin, enoxacin and the like; monobactam, penem and carbapenem antibiotics such as carumonam sodium and the like. Examples of chemotherapeutics include sulfamethizole and the like.

Examples of antidiabetic agents include tolbutamide, vogilibose, pioglitazone hydrochloride, glibenclamide, troglitazone, rosiglitazone and the like. Examples of antispasmodics include meclizine hydrochloride, dimenhydrinate and the like. Examples of antirheumatic drugs include methotrexate, bacillamine and the like. Examples of hormono drugs include liothyronine sodium, dexamethasone sodium phosphate, prednisolone, oxendolone, leuproreilin acetate and the like. Examples of alkaloidal narcotics include opium, morphine hydrochloride, thoron, oxycodone, opium alkaloids hydrochloride, cocaine hydrochloride and the like. Examples of sulfa drugs include sulfisomidine, sulfamethizole and the like. Examples of drugs for treatment of gout include allopurinol, colchicine and the like. Examples of anticoagulants include dicoumarol and the like. Examples of antineoplastics include 5-fluorouracil, uracil, mitomycin, manidipine hydrochloride, voglibose, candesartan cilexetil, pioglitazone hydrochloride and the like.

Examples of other active ingredients include tamsulosin hydrochloride, donepezile hydrochloride, oseltamivir, limaprost alfadex, loxoprofen sodium, sarpogrelate hydrochloride, ursodeoxycholic acid, alacepril, brotizolam, berberine hydrochloride or tannate, loperamide hydrochloride, ebastine, alfacalcidol, betamethasone, cabergoline, resedronate sodium, raloxifene hydrochloride, alendronate sodium, nicergoline, isosorbide dinitrate, taltirelin, naftopidil, granisetron hydrochloride, camostat mesilate, tacrolimus, clopidogrel, alendronate sodium hydrate, imatinib mesylate, sodium risedronate hydrate and the like.

As nutritional components, proteins, saccharides, lipid, vitamins, minerals, other effective components are taken. Example of vitamins include astaxanthin, vitamin A, carotenoids such as β -carotene, lutein, zeaxanthin and the like, vitamin Bi, its derivatives, or salts thereof such as fursultiamine, fursultiamine hydrochloride, prosultiamine, octotiamine, tiaminedisulfide, bisbentiamine, bisbutythiamin, bisibutiamine, benfotiamine, cetotiamine hydrochloride and the like; vitamin B₂, its derivatives or salts thereof such as riboflavin, sodium riboflavin phosphate, sodium flavin-adenine-dinucleotide, riboflavin butyrate and the like; vitamin C or its derivatives such as ascorbic acid, ascorbic acid glucoside, L-ascorbyl palmitate, L-ascorbyl phosphate and the like; vitamin E substances such as tocopherol, tocopherol acetate, tocopherol succinate, tocopherol nicotinate, tocotrienol and the like.

Examples of other active ingredients include deoxyribo nucleic acid and its salt, adenylic acid derivatives such as adenosine triphosphate, adenosine monophosphate and salts thereof; nucleic acid related substances such as ribonucleic acid and its salt, guanine, xanthine, derivatives thereof and salts thereof; extracts derived from animals such as extract of deproteinized serum, spleen extract, placenta extract, crest extract, royal jerry and the like; extracts derived from microorganisms such as yeast extract, extract of lactic acid bacteria, extract of bifid bacteria and the like; extracts derived from plants such as *Fomes Japonioucus* extract, carrot extract, *Swertia* extract, rosemary extract, phellodendron bark extract, garlic extract, Hinokitiol, cepharanthine, aloe extract, *Salvia splendens* extract, arunica extract, camonile extract, white birch extract, *hypericum* extract, *Eucalyptus* extract, *Xuan Fu Hua* extract, *Luffa* extract, *Cnidium officinale* extract, ginseng extract, blueberry extract, ginkgo leaf extract, *patholobus suberectus Dunn* extract, Sanpenzu extract, moricortex extract, *Angerica* extract, *Bistorta* extract, *Sophora* extract, *Crataegus* extract, white lily extract, hop extract, wild rose extract, *Coix lacryma-jobi* extract and the like; α- or γ-linolenic acid, eicosapentaenoic acid and derivatives thereof: succinic acid, its derivatives, and salts thereof; estradiol, its derivatives and salts thereof; α- hydroxy acids such as glycolic acid, lactic acid, malic acid, citric acid, salicylic acid and the like, derivatives thereof and salts thereof; glycyrrhzic acid, glycyrrhetic acid, mefenamic acid, phenylbutazone, indomethacin, ibuprofen, ketoprofen, allantoin, guaiazulene, their derivatives and their salts; cysteine, its derivatives and salts thereof; hyaluronic acid, chondroitin, chondroitin sulfuric acid, dermatan sulfate, heparan sulfate, heparin, keratan sulfate and their salts; collagen, elastin, keratin, their derivatives and salts; ε-aminocaproic acid, deep ocean water, powdered papaya, zinc oxide, zinc, DHA, glutatione, flavonoid, tannin, ellagic acid, nucleic acids, herbal medicines, seaweeds, inorganic substances, and mixture thereof.

In the case that the effective ingredient has bitterness or it is intended so as to release in digestitive tract, there can be used one treated by the known method such as coating method. For example, according to the method described in JP 11-263723 A, the effective ingredient may be coated with a water-soluble saccharide such as xylitol, sorbitol or sucrose, a water soluble binding agent such as polyvinyl pyrrolidone, pullulan, hydroxypropylcellulose, hydroxypropylmethylcellulose, gum arabic or gelatin, and an excipient such as mannitol, lactose or mannose by spray-drying, fluidized bed granulation, agitated granulation or kneading granulation. The effective ingredient may be also coated with a gelling agent, a binding agent and sugar alcohol according to the method described in JP 2002-275054 A.

If an astringency, sourness or bitterness, each derived from the raw material is such extent that they can be suppressed by seasoning or flavoring, there can be incorporated an acidifier (e.g., citric acid, tartaric acid, malic acid, ascorbic acid or the like), a sweetener (e.g., accharine sodium, dipotassium glycyrrhizinate, aspartame, stevia, thaumatin or the like), a corrigent and a perfume (e.g., a variety of fruit fragrances including lemon oil, orange oil, strawberry; yoghrut, peppermint, menthol or the like). These seasoning or flavoring agents may be incorporated in an amount of 0.01 to 0.5 parts by weight based on 100 parts by weight of the whole tablet.

There can be incorporated into the composition to be compression-molded other ingredients which do not injure the disintegrating property of the molded material and which can be incorporated in the pharmaceuticals. Examples of such ingredients are a surfactant (e.g., polyoxyethylene hydrogenated caster oil, polyoxyethylene polyoxypropylene glycol, sorbitan fatty acid ester, polysorbate, glycerin fatty acid ester, sodium lauryl sulfate or the like), an effervescent agent (e.g. sodium hydrogen carbonate, sodium carbonate or the like), a coloring agent ((e.g., food red No. 2, food blue No.2, food yellow No. 5, food lake pigment, ferric oxide, carmine or the like), a stabilizer (e.g., sodium edatate, tocopherol, cyclodextrin, or the like), etc.

The rapid disintegrating compression-molded material may be prepared by mixing together the particle composition of the present invention, at least one selected from among a lubricant, an excipient, a disintegrant aid and/or a binder aid, an effective agent and other ingredient which can be incorporated in the pharmaceuticals and compression-molding the resultant mixture. It is preferable that compression molding is performed by a direct tableting method. The tableting pressure under tableting is varied depending on the size of tablet, but it is usually 200-2000kgf, preferably 250-1600 kgf, more preferably 250-1200 kgf.

The rapid disintegrating compression-molded material of the present invention has usually hardness of 10-200N, preferably 20-100N. Since it contains the particle composition of the present invention, application of low tableting pressure provides a good hardness. The tableting pressure is varied depending on the size of tablet. For example, in tableting a tablet weighing 200mg using a pastle having 8 mm of diameter, the application of tableting pressure of 100-1200 kgf provides hardness of 20-150 N, and the application of tableting pressure of 100-1000 kgf provides hardness of 20-80 N.

As other preparation process, the particle composition of the present invention, at least one selected from among a lubricant, an excipient, a disintegrant aid and/or a binder aid, and an effective agent may be wet granulated and thereafter compression molded. Examples of wet-granulating process are a spray drying process, a fluidized bed granulated drying process, an agitated granulating process and wet extruding granulating process. Also, after the compression molding has been conducted, an aging such as heating or humidification may be conducted according to the conventional process thereby the desired hardness and disintegrating property may be imparted into the compression-molded material.

In preparing the rapid disintegrating compression-molded material of the present invention, a lubricant may be mixed with other ingredients and compression-molded as mentioned in the above, but it may be previously applied onto the surface of a pestle and the wall surface of mortar of a compression-molding machine without mixing with other ingredients, and subjected to compression molding (external lubricating method) whereby the desired hardness and disintegrating property may be imparted into the compression-molded material. The application process of a lubricant onto a pestle and a mortar may be conducted by the method hitherto known or using a machine.

The rapid disintegrating compression-molded material of the present invention may also be used as other solid preparation than the intended rapid disintegrating property, such as chewable tablet. In preparing chewable tablet, lower hardness than that as aforementioned may be set.

Since the rapid disintegrating compression-molded material of the present invention disintegrates immediately in a small amount of water, it may be used not only as pharmaceuticals but as foods or health foods, Food for Specified Health Uses, pet foods, feeds and agricultural chemicals.

### Examples

The invention will be explained hereinafter with reference to Examples but these Examples do not restrict the range of the present invention.

The evaluation for the respective tablets obtained in Examples was made according to the following manners:

### [Disintegration time in the oral cavity]

Each of 3-8 persons to be tested placed a tablet (every one tablet, n=6) on the tongue in the oral cavity and measured the time required for the tablet to be disintegrated completely. The average value was regarded as the disintegration time.

### [Sensory test]

Each of 3-8 persons to be tested placed a tablet on the tongue in the oral cavity to disintegrate spontaneously. After-taste was confirmed and the tablet which the greater part of the persons to be tested recognized to be good was evaluated as "good".

### [Hardness of the tablet]

The hardness was measured using a load-cell type tablet hardness tester ("PC-30", a product of OKADA SEIKO CO., LTD.).

### [Tableting property]

A pestle and a mortal of the tableting machine and the tablet after tableting were observed to evaluate capping and sticking.

### [Example 1]: Preparation of a particle composition

5 Parts by weight of xylitol, 64 parts by weight of corn starch, 15 parts by weight of waxy corn starch, 8.5 parts by weight of crystalline cellulose, 5 parts by weight of light magnesium carbonate and 2.5 parts by weight of light calcium carbonate were dispersed uniformly in water and thereafter subjected to spray-drying at an outlet temperature of 90-100 °C using a spray drying machine ("S-50N/R", a product of Niro Inc.) to obtain white and spherical particles composition having a good flowability.

### [Example 2]: Preparation of a particle composition

5 Parts by weight of lactose, 57.5 parts by weight of corn starch, 15 parts by weight of rice starch ("Micro pearl", a product of Shimada Chemical Industry Co., Ltd.), 15 parts by weight of crystalline cellulose, 5 parts by weight of light magnesium carbonate and 2.5 parts by weight of light calcium carbonate were dispersed uniformly in water and thereafter subjected to spray-drying at an outlet temperature of 90-100 °C using a spray drying machine ("S-50N/R", a product of Niro Inc.) to obtain white and spherical particles composition having a good flowability.

### [Example 3]: Preparation of a particle composition

10 Parts by weight of granulated sugar, 70 parts by weight of corn starch and 20 parts by weight of magnesium carbonate were dispersed uniformly in water and thereafter subjected to spray-drying at an outlet temperature of 90-100 °C using a spray drying machine ("L-8 type", a product of Ohkawara Kakohki Co., Ltd.) to obtain white and spherical particles composition having a good flowability.

### [Example 4]: Preparation of a particle composition

10 Parts by weight of xylitol, 70 parts by weight of corn starch and 20 parts by weight of magnesium carbonate were dispersed uniformly in water and thereafter subjected to spray-drying at an outlet temperature of 90-100 °C using a spray drying machine ("L-8 type", a product of Ohkawara Kakohki Co., Ltd.) to obtain white and spherical particles composition having a good flowability.

### [Example 5]: Preparation of a particle composition

49 Parts by weight of corn starch, 30 parts by weight of waxy corn starch, 8.5 parts by weight of microcrystalline cellulose, 5 parts by weight of xylitol, 5 parts by weight of magnesium carbonate and 2.5 parts by weight of light calcium carbonate were dispersed uniformly in water and thereafter subjected to spray-drying at an outlet temperature of 90-100 °C using a spray drying machine ("L-8 type", a product of Ohkawara Kakohki Co., Ltd.) to obtain white and spherical particles composition having a good flowability.

### [Example 6]: Preparation of a particle composition

49 Parts by weight of corn starch, 8.5 parts by weight of microcrystalline cellulose, 5 parts by weight of xylitol, 5 parts by weight of magnesium carbonate and 2.5 parts by weight of light calcium carbonate were dispersed uniformly in water and thereafter subjected to spray-drying at an outlet temperature of 90-100 °C using a spray drying machine ("S-50N/R", a product of Niro Inc.) to obtain white and spherical particles composition having a good flowability. SEM photograph of this particle was shown in figure 1.

### [Comparative Example 1]: Preparation of rapid disintegrating compression-molded material by dry blending

5 Parts by weight of xylitol, 64 parts by weight of corn starch, 15 parts by weight of waxy corn starch, 8.5 parts by weight of crystalline cellulose, 5 parts by weight of light magnesium carbonate and 2.5 parts by weight of light calcium carbonate were placed in a plastic bag and shaken well to obtain a mixture. After 99.8 parts by weight of this granulated matter was mixed with 0.2 part by weight of glyceryl behenate ("Compritol 888ATO", a product of Gattefosse S.A.S.), an attempt was made to obtain tablets, each weighing 200 mg, having a diameter of 8 mm and 9R by tableting the resultant mixture under setting hardness of 30 N using a rotary tableting machine. As a result, the mixture could not be filled uniformly into a mortar and tableting procedure could not be conducted owing to high bulk density.

### [Comparative Example 2]: Preparation of rapid disintegrating compression-molded material by dry blending

10 Parts by weight of granulated sugar, 70 parts by weight of corn starch and 20 parts by weight of magnesium carbonate and were was placed in a plastic bag and shaken well to obtain a mixture. 94 Parts by weight of this mixture, 2 parts by weight of CMC-Ca, 3 parts by weight of sugar ester and 1 part by weight of silicon dioxide ("Sylopage" a product of Fuji Silysia Chemical Ltd.) were mixed together, and thereafter an attempt was made to obtain tablets, each weighing 200 mg, having a diameter of 8 mm and 9R by tableting the resultant mixture under setting hardness of 30 N using a rotary tableting machine. As a result, the mixture could not be filled uniformly into a mortar and tableting procedure could not be conducted owing to high bulk density.

### [Example 7]: Preparation of rapid disintegrating compression-molded material

99.8 Parts by weight of granulated matter prepared in Example 1 and 0.2 parts by weight of glyceryl behenate ("Compritol 888ATO" , a product of Gattefosse S.A.S.) were mixed together, and thereafter the mixture was tableted as a setting hardness of 30 N using a rotary tableting machine to obtain tablets each weighing 200 mg, having a diameter of 8 mm and 9R.

### [Example 8]: Preparation of rapid disintegrating compression-molded material

99.8 Parts by weight of granulated matter prepared in Example 2 and 0.2 parts by weight of glyceryl behenate ("Compritol 888ATO" , a product of Gattefosse S.A.S.) were mixed together, and thereafter the mixture was tableted as a setting hardness of 30 N using a rotary tableting machine to obtain tablets each weighing 200 mg, having a diameter of 8 mm and 9R.

### [Example 9]: Preparation of rapid disintegrating compression-molded material

94 Parts by weight of granulated matter prepared in Example 1, 2 parts by weight of CMC-Ca, 3 parts by weight of sugar ester and 1 part by weight of silicon dioxide ("Sylopage" a product of Fuji Silysia Chemical Ltd.) were mixed together, and thereafter the mixture was tableted as a setting hardness of 30 N using a rotary tableting machine to obtain tablets each weighing 200 mg, having a diameter of 8 mm and 9R.

### [Example 10]: Preparation of rapid disintegrating compression-molded material

94 Parts by weight of granulated matter prepared in Example 3, 2 parts by weight of CMC-Ca, 3 parts by weight of sugar ester and 1 part by weight of silicon dioxide ("Sylopage" a product of Fuji Silysia Chemical Ltd.) were mixed together, and thereafter the mixture was tableted as a setting hardness of 30 N using a rotary tableting machine to obtain tablets each weighing 200 mg, having a diameter of 8 mm and 9R.

### [Example 11]: Preparation of rapid disintegrating compression-molded material

64.68 Parts by weight of granulated matter prepared in Example 1, 2 parts by weight of CMC-Ca, 3 parts by weight of sugar ester and 1 part by weight of silicon dioxide ("Sylopage" a product of Fuji Silysia Chemical Ltd.), 27.72 parts by weight of corn starch, 0.1 part by weight of aspartame and 1.5 part by weight of menthol were mixed together, and thereafter the mixture was tableted as a setting hardness of 30 N using a rotary tableting machine to obtain tablets each weighing 200 mg, having a diameter of 8 mm and 9R.

### [Example 12]: Preparation of rapid disintegrating compression-molded material

64.68 Parts by weight of granulated matter prepared in Example 3, 2 parts by weight of CMC-Ca, 3 parts by weight of sugar ester and 1 part by weight of silicon dioxide ("Sylopage" a product of Fuji Silysia Chemical Ltd.), 27.72 parts by weight of corn starch, 0.1 part by weight of aspartame and 1.5 part by weight of menthol were mixed together, and thereafter the mixture was tableted as a setting hardness of 30 N using a rotary tableting machine to obtain tablets each weighing 200 mg, having a diameter of 8 mm and 9R.

### [Example 13]: Preparation of rapid disintegrating compression-molded material

99.6 Parts by weight of granulated matter prepared in Example 5 was mixed with 0.4 part by weight of glyceryl behenate, and thereafter the mixture was tableted as a setting hardness of 50 N using a rotary tableting machine to obtain tablets each weighing 200 mg, having a diameter of 8 mm and 9R.

### [Example 14]: Preparation of rapid disintegrating compression-molded material

70 Parts by weight of granulated matter prepared in Example 6 was mixed with 30 parts by weight of corn starch, and 99.6 parts by weight of this mixture was mixed with 0.4 part by weight of glyceryl behenate, and thereafter the resultant mixture was tableted as a setting hardness of 50 N using a rotary tableting machine to obtain tablets each weighing 200 mg, having a diameter of 8 mm and 9R.

### [Example 15]: Preparation of rapid disintegrating compression-molded material

70 Parts by weight of granulated matter prepared in Example 6 was mixed with 30 parts by weight of waxy corn starch, and 89.6 parts by weight of this mixture, 10 parts by weight of acetaminophen and 0.4 part by weight of glyceryl behenate were mixed together, and thereafter the resultant mixture was tableted as a setting hardness of 50 N using a rotary tableting machine to obtain tablets each weighing 200 mg, having a diameter of 8 mm and 9R.

### [Example 16]: Preparation of rapid disintegrating compression-molded material

10 Parts by weight of acetaminophen, 49 parts by weight of corn starch, 8.5 parts by weight of microcrystalline cellulose, 5 parts by weight of xylitol, 5 parts by weight of magnesium carbonate and 2.5 parts by weight of calcium carbonate were dispersed uniformly in water, and thereafter subjected to spray-drying at an outlet temperature of 90-100 °C using a spray drying machine machine ("L-8 type", a product of Ohkawara Kakohki Co., Ltd.) to obtain a spherical particle composition having a good flowability.

68 Parts by weight of this granulated matter, 29.2 parts by weight of corn starch, 0 . 2 part by weight of aspartame, 0 . 2 part by weight of a sweetener acesulfame potassium ("Sunett D" a product of Kirin Kyowa Foods Company), 2 parts by weight of orange flavor and 0.4 part by weight of glyceryl behenate were mixed together, and thereafter the resultant mixture was tableted as a setting hardness of 50 N using a rotary tableting machine to obtain tablets each weighing 200 mg, having a diameter of 8 mm and 9R.

### [Example 17]: Preparation of rapid disintegrating compression-molded material

60 Parts by weight of corn starch, 17.5 parts by weight of glycine, 15 parts by weight of microcrystalline cellulose, 5 parts by weight of magnesium carbonate and 2.5 parts by weight of calcium carbonate were dispersed uniformly in water, and thereafter subjected to spray-drying at an outlet temperature of 90-100 °C using a spray drying machine ("L-8 type", a product of Ohkawara Kakohki Co., Ltd.) to obtain a spherical particle composition having a good flowability.

99.8 Parts by weight of this granulated matter and 0 . 2 part by weight of glyceryl behenate ("Compritol 888ATO" were mixed together, and thereafter the resultant mixture was tableted as a setting hardness of 30 N using a rotary tableting machine to obtain tablets each weighing 200 mg, having a diameter of 8 mm and 9R.

**[Table 1]: Results of tests**

| | Hardness [N] | Disintegration Time [sec] | Tableting Property | Taking feeling |
|---|---|---|---|---|
| Comparative Example 1 | - | - | Tableting was impossible | - |
| Comparative Example 2 | - | - | Tableting was impossible | - |
| Example 7 | 20.6 | 12.3 | Good | Good |
| Example 8 | 31.0 | 14.9 | Good | Good |
| Example 9 | 36.5 | 32.0 | Good | Good |
| Example 10 | 20.7 | 28.9 | Good | Good |
| Example 11 | 27.3 | 22.2 | Good | Good |
| Example 12 | 43.3 | 26.6 | Good | Good |
| Example 13 | 30.6 | 25.6 | Good | Good |
| Example 14 | 52.0 | 11.9 | Good | Good |
| Example 15 | 53.1 | 18.0 | Good | Good |
| Example 16 | 52.4 | 15.2 | Good | Good |
| Example 17 | 32.8 | 39.6 | Good | Good |

As shown by Comparative Examples 1 and 2, in the case that the composition of the present invention was subjected to dry mixing, it could not filled uniformly so that the tablecting procedure could not be performed. Contrary thereto, it is apparent from the Examples 7-16 that the tablets obtained by subjecting the ingredients involving in the present invention to wet granulation and tableting the resultant particle composition have good hardness, disintegration time in the oral cavity, tableting property and taking property. This indicates that the disintegrating particle composition has good characteristics as an excipient for rapid disintegrating compression-molded material.

### [Pharmaceutical Preparation Example]: rapid disintegrating compression-molded material

60 Parts by weight of corn starch, 17.5 parts by weight of DL-alanine, 15 parts by weight of microcrystalline cellulose, 5 parts by weight of magnesium carbonate and 2.5 parts by weight of calcium carbonate were dispersed uniformly in water, and thereafter subjected to spray-drying at an outlet temperature of 90-100 °C using a spray drying machine ("L-8 type", a product of Ohkawara Kakohki Co., Ltd.) to obtain a spherical particle composition having a good flowability. Consequently, 99.8 parts by weight of this granulated matter, 0 . 2 part by weight of glyceryl behenate (behenate ("Compritol 888ATO" , a product of Gattefosse S.A.S.) were mixed together, and thereafter the resultant mixture was tableted as a setting hardness of 30 N using a rotary tableting machine to obtain tablets each weighing 200 mg, having a diameter of 8 mm and 9R.

## Claims

1. A disintegrating particle composition whose average particle size is 1-500 µm and wherein a water-insoluble starch and a water-soluble binding agent are present in the ratio by weight of 1:0.01-1:0.75 and in the spherical form of complex particles, said starch being in a form of particles and being singly and uniformly dispersed in the matrix of the water-soluble binding agent,
wherein said water-insoluble starch is a starch derived from grain, bean, tuber or root of plant,
wherein said water-soluble binding agent is saccharide or saccharide alcohol or amino acid, and
wherein said saccharide or saccharide alcohol is a member selected from the group consisting of xylitol, erythritol, maltitol, sorbitol, mannitol, lactose, sucrose, glucose, fructose, maltitol, trehalose and palatinose.

2. The disintegrating particle composition as claimed in claim 1 wherein the starch and the water-soluble binding agent are present in the ratio by weight of 1:0.03-0.3.

3. The disintegrating particle composition as claimed in claim 1 or 2 wherein the starch is a member selected from the group consisting of corn starch, rice starch, potato starch, wheat starch, glutinous rice starch, waxy corn starch, sweet potato starch, tapioca starch and taro starch.

4. The disintegrating particle composition as claimed in claim 1 or 2 wherein the amino acid is a member selected from the group consisting of glycine, alanine, serine, lysine, threonine, cysteine, hystidine, asparagines, aspartic acid, glutamine and glutamic acid.

5. The disintegrating particle composition as claimed in any one of claims 1 to 4 wherein a molding agent is further contained.

6. The disintegrating particle composition as claimed in claim 5 wherein the ratio by weight of the starch to the molding agent is in the range of 1:0.01-1.

7. The disintegrating particle composition as claimed in claim 5 wherein the ratio by weight of the starch to the molding agent is in the range of 1:0.1-0.4.

8. The disintegrating particle composition as claimed in any one of claims 5-7 wherein the molding agent is crystalline cellulose or inorganic particles.

9. The disintegrating particle composition as claimed in claim 8 wherein ratio by weight of the molding agent to inorganic particles is in the range of 1:0.06-10.

10. The disintegrating particle composition as claimed in claims 8 or 9 wherein inorganic particles are selected from the group consisting of magnesium carbonate, calcium carbonate, calcium phosphate, calcium hydrogen phosphate, anhydrous calcium hydrogen phosphate, magnesium aluminomethasilicate, magnesium aluminosilicate, hydrotalcite, aluminum silicate, calcium silicate, magnesium silicate, anhydrous silicic acid, silicon dioxide, magnesium oxide, magnesium hydroxide, magnesia alumina hydrate, dry aluminum hydroxide gel crystalline cellulose and powdered cellulose.

11. The disintegrating particle composition as claimed in any one of claims 1 to 10 wherein an effective ingredient is further contained.

12. A process for preparing a disintegrating particle composition claimed in any one of claims 1 to 11 wherein said disintegrating particle composition is obtained by subjecting a water-insoluble starch, a water-soluble binding agent, and if needed a molding agent and/or an effective ingredient to wet granulation.

13. The process for preparing a disintegrating particle composition as claimed in claim 12 wherein wet granulation is spray-drying process.

14. A rapid disintegrating compression-molded material containing a disintegrating particle composition claimed in any one of claims 1 to 11.

15. A rapid disintegrating compression-molded material which comprises incorporated 0.01-1000 parts by weight of the ingredient selected from the following group, and if needed 0.01-100 parts by weight of an effective ingredient based on 100 parts by weight of the disintegrating particle composition claimed in any one of claims 1 to 11:
An excipient, disintegrating agent, a binding agent, a surfactant, a lubricant, an acidifier, a sweetener, a corrigent and a perfume, a coloring agent and a stabilizer.

## Patentansprüche

1. Zerfallende Partikelzusammensetzung, deren durchschnittliche Partikelgröße 1 - 500 µm ist und in der eine wasserunlösliche Stärke und ein wasserlösliches Bindemittel im Gewichtsverhältnis von 1:0,01 - 1:0,75 und in der sphärischen Form von komplexen Partikeln vorliegen, wobei die Stärke in der Form von Partikeln ist und diese einzeln und gleichmäßig in der Matrix des wasserlöslichen Bindemittels dispergiert sind,
wobei die wasserunlösliche Stärke eine Stärke ist, die aus Pflanzenkorn, -bohne, -knolle oder -wurzel stammt,
wobei das wasserlösliche Bindemittel Saccharid oder Saccharidalkohol oder Aminosäure ist und
wobei das Saccharid oder der Saccharidalkohol ein Mitglied, ausgewählt aus der Gruppe, bestehend aus Xylit, Erythritol, Maltit, Sorbit, Mannit, Lactose, Saccharose, Glucose, Fructose, Maltit, Trehalose und Palantinose, ist.

2. Zerfallende Partikelzusammensetzung gemäß Anspruch 1, wobei die Stärke und das wasserlösliche Bindemittel im Gewichtsverhältnis von 1:0,03 - 0,3 vorhanden sind.

3. Zerfallende Partikelzusammensetzung gemäß Anspruch 1 oder 2, wobei die Stärke ein Mitglied, ausgewählt aus der Gruppe, bestehend aus Maisstärke, Reisstärke, Kartoffelstärke, Weizenstärke, glutenreicher Reisstärke, Wachsmaisstärke, Süßkartoffelstärke, Tapiokastärke und Tarostärke, ist.

4. Zerfallende Partikelzusammensetzung gemäß Anspruch 1 oder 2, wobei die Aminosäure ein Mitglied, ausgewählt aus der Gruppe, bestehend aus Glycin, Alanin, Serin, Lysin, Threonin, Cystein, Histidin, Asparagin, Asparaginsäure, Glutamin und Glutaminsäure, ist.

5. Zerfallende Partikelzusammensetzung gemäß einem der Ansprüche 1 bis 4, wobei außerdem ein Formungsmittel enthalten ist.

6. Zerfallende Partikelzusammensetzung gemäß Anspruch 5, wobei das Gewichtsverhältnis der Stärke zu dem Formungsmittel im Bereich von 1:0,01 - 1 liegt.

7. Zerfallende Partikelzusammensetzung gemäß Anspruch 5, wobei das Gewichtsverhältnis der Stärke zu dem Formungsmittel im Bereich von 1:0,1 - 0,4 liegt.

8. Zerfallende Partikelzusammensetzung gemäß einem der Ansprüche 5 - 7, wobei das Formungsmittel kristalline Cellulose oder anorganische Partikel ist.

9. Zerfallende Partikelzusammensetzung gemäß Anspruch 8, wobei das Gewichtsverhältnis des Formungsmittels zu anorganischen Partikeln im Bereich von 1:0,06 - 10 liegt.

10. Zerfallende Partikelzusammensetzung gemäß Anspruch 8 oder 9, wobei anorganische Partikel aus der Gruppe, bestehend aus Magnesiumcarbonat, Calciumcarbonat, Calciumphosphat, Calciumhydrogenphosphat, wasserfreiem Calciumhydrogenphosphat, Magnesiumaluminometasilikat, Magnesiumaluminosilikat, Hydrotalcit, Aluminiumsilikat, Calciumsilikat, Magnesiumsilikat, wasserfreier Kieselsäure, Siliciumdioxid, Magnesiumoxid, Magnesiumhydroxid, Magnesiumoxid-Aluminiumoxid-Hydrat, trockenem Aluminiumhydroxidgel, kristalliner Cellulose und pulverförmiger Cellulose, ausgewählt sind.

11. Zerfallende Partikelzusammensetzung gemäß einem der Ansprüche 1 bis 10, wobei außerdem ein wirksames Ingredienz enthalten ist.

12. Verfahren zur Herstellung einer zerfallenden Partikelzusammensetzung gemäß einem der Ansprüche 1 bis 11, wobei die zerfallende Partikelzusammensetzung erhalten wird, indem eine wasserunlösliche Stärke, ein wasserlösliches Bindemittel und, falls erforderlich, ein Formungsmittel und/oder ein wirksames Ingredienz einer Nassgranulierung unterzogen werden.

13. Verfahren zur Herstellung einer zerfallenden Partikelzusammensetzung gemäß Anspruch 12, wobei Nassgranulierung ein Sprühtrocknungsverfahren ist.

14. Ein rasch zerfallendes kompressionsgeformtes Material, enthaltend eine zerfallende Partikelzusammensetzung gemäß einem der Ansprüche 1 bis 11.

15. Rasch zerfallendes kompressionsgeformtes Material, das 0,01 - 1 000 Gewichtsteile des Ingredienz, ausgewählt aus der folgenden Gruppe, und, falls erforderlich, 0,01 - 100 Gewichtsteile eines wirksamen Ingredienz, basierend auf 100 Gewichtsteilen der zerfallenden Partikelzusammensetzung gemäß einem der Ansprüche 1 bis 11 eingearbeitet umfasst:
ein Excipiens, ein Zerfallsmittel, ein Bindemittel, ein Surfactant, ein Schmiermittel, ein Säuerungsmittel, ein Süßungsmittel, ein Korrigens und ein Parfüm, ein Färbemittel und ein Stabilisator.

## Revendications

1. Composition de particule de désintégration dont la taille moyenne de particule est de 1-500 µm et dans laquelle un amidon insoluble dans l'eau et un agent liant soluble dans l'eau sont présents dans le rapport en masse de 1:0,01-1:0,75 et dans la forme sphérique de particules complexes, ledit amidon étant dans une forme de particules et étant dispersé seul et uniformément dans la matrice de l'agent liant soluble dans l'eau,
dans laquelle ledit amidon insoluble dans l'eau est un amidon dérivé de grain, fève, tubercule ou racine de plante,
dans laquelle ledit agent liant soluble dans l'eau est un saccharide ou alcool de saccharide ou acide aminé, et
dans laquelle ledit saccharide ou alcool de saccharide est un élément choisi dans le groupe consistant en xylitol, érythritol, maltitol, sorbitol, mannitol, lactose, saccharose, glucose, fructose, maltitol, tréhalose et palatinose.

2. Composition de particule de désintégration selon la revendication 1, dans laquelle l'amidon et l'agent liant soluble dans l'eau sont présents dans le rapport en masse de 1:0,03-0,3.

3. Composition de particule de désintégration selon la revendication 1 ou 2, dans laquelle l'amidon est un élément choisi dans le groupe consistant en amidon de maïs, amidon de riz, amidon de pomme de terre, amidon de blé, amidon de riz glutineux, amidon de maïs cireux, amidon de pomme de terre douce, amidon de tapioca et amidon de taro.

4. Composition de particule de désintégration selon la revendication 1 ou 2, dans laquelle l'acide aminé est un élément choisi dans le groupe consistant en glycine, alanine, sérine, lysine, thréonine, cystéine, histidine, asparagines, acide aspartique, glutamine et acide glutamique.

5. Composition de particule de désintégration selon l'une quelconque des revendications 1 à 4, dans laquelle un agent moulant est de plus contenu.

6. Composition de particule de désintégration selon la revendication 5, dans laquelle le rapport en masse de l'amidon à l'agent de moulage se trouve dans l'intervalle de 1:0,01-1.

7. Composition de particule de désintégration selon la revendication 5, dans laquelle le rapport en masse de l'amidon à l'agent de moulage se trouve dans l'intervalle de 1:0,1-0,4.

8. Composition de particule de désintégration selon l'une quelconque des revendications 5-7, dans laquelle l'agent moulant est de la cellulose cristalline ou des particules inorganiques.

9. Composition de particule de désintégration selon la revendication 8, dans laquelle le rapport en masse de l'agent moulant aux particules inorganiques se trouve dans l'intervalle de 1:0,06-10.

10. Composition de particule de désintégration selon la revendication 8 ou 9, dans laquelle les particules inorganiques sont choisies dans le groupe consistant en carbonate de magnésium, carbonate de calcium, phosphate de calcium, hydrogénophosphate de calcium, hydrogénophosphate de calcium anhydre, aluminométasilicate de magnésium, aluminosilicate de magnésium, hydrotalcite, silicate d'aluminium, silicate de calcium, silicate de magnésium, acide silicique anhydre, dioxyde de silicium, oxyde de magnésium, hydroxyde de magnésium, hydrate de magnésie alumine, cellulose cristalline de gel d'hydroxyde d'aluminium sèche et cellulose en poudre.

11. Composition de particule de désintégration selon l'une quelconque des revendications 1 à 10, dans laquelle un ingrédient efficace est de plus contenu.

12. Procédé de préparation d'une composition de particule de désintégration selon l'une quelconque des revendications 1 à 11, dans lequel ladite composition de particule de désintégration est obtenue en soumettant un amidon insoluble dans l'eau, un agent liant soluble dans l'eau, et si nécessaire un agent de moulage et/ou un ingrédient efficace à une granulation par voie humide.

13. Procédé de préparation d'une composition de particule de désintégration selon la revendication 12, dans lequel une granulation par voie humide est un procédé de séchage par pulvérisation.

14. Matériau moulé par compression se désintégrant rapidement contenant une composition de particule de désintégration selon l'une quelconque des revendications 1 à 11.

15. Matériau moulé par compression se désintégrant rapidement qui comprend incorporées 0,01-1 000 parties en masse de l'ingrédient choisi dans le groupe suivant, et si nécessaire 0,01-100 parties en masse d'un ingrédient efficace sur la base de 100 parties en masse de la composition de particule de désintégration selon l'une quelconque des revendications 1 à 11 : un excipient, un agent désintégrant, un agent liant, un tensioactif, un lubrifiant, un acidifiant, un édulcorant, un correcteur et un parfum, un agent colorant et un stabilisant.
